# EUROPEAN PATENT SPECIFICATION

(11) **EP 1 765 371 B1**
(45) Date of publication and mention of the grant of the patent: **08.08.2012**
(21) Application number: 05747206.0
(22) Date of filing: 15.04.2005
(51) Int. Cl.: A61K 36/185, A23K 1/14, A23K 1/16, A23K 1/18, A61K 36/63, A61K 36/28, A61K 36/61, A61K 36/76, A61K 36/736, A61K 36/355, A61K 36/515, A61K 36/65, A61K 36/708, A61K 36/45, A61K 36/30

(54) **USE OF PLANTS AND PLANT EXTRACTS AS FEED ADDITIVE TO AFFECT THE RUMEN FERMENTATION**
VERWENDUNG VON PFLANZEN UND PFLANZENEXTRAKTEN ALS FUTTERMITTELZUSATZ ZUR BEEINFLUSSUNG DER RUMEN-FERMENTIERUNG
UTILISATION DE PLANTES, D'EXTRAITS DE PLANTES ET DE COMPOSANTS IDENTIQUES OU PRODUITS NATURELS DE PLANTES AFIN DE MODIFIER LA FERMENTATION DU RUMEN

(30) Priority: 16.04.2004 SE 0400996
(43) Date of publication of application: 28.03.2007
(73) Proprietor: DSM IP Assets B.V., 6411 TB Heerlen (NL)
(72) Inventor: BECKER, Klaus, 72622 Nürtingen-Reudern (DE); DUFFY, Cepta, D., Dublin, 15 (IE); HOFFMANN, Ellen, 72072 Tübbingen (DE); LOSA, Riccardo, CH-1145 Bière (CH); MOULD, Fergus, Laurence, Reading RG6 7JX (GB); MUETZEL, Stefan, Aberdeen AB21 9SF (GB); SECUNDINO, López, Puente, E-24007 Le n (ES); SELJE, Natascha, 70794 Filderstadt (DE); WALLACE, Robert, John, Aberdeen AB32 6YF (GB)
(74) Representative: Schwander, Kuno
(86) International application number: PCT/EP2005/051673
(87) International publication number: WO 2005/099729

(56) References cited:
- EP-A- 0 761 103
- EP-A- 1 297 751
- WO-A-03/094628
- RANDHAWAR, S.S. ET AL.: "Effect of herbal biostimulators on biochemical constituents of rumen liquor, blood and milk in relation to milk production in cows" INDIAN JOURNAL OF INDIGENOUS MEDICINES, vol. 16, no. 2, 1995, pages 73-92, XP008052953
- HOFFMANN, E.M. ET AL.: "Effects of Moringa oleifera seed extract on rumen fermentation in vitro" ARCHIVES OF ANIMAL NUTRITION, vol. 57, no. 1, February 2003 (2003-02), pages 65-81, XP008052920 cited in the application
- GUPTA, N. ET AL.: "Effect of herbs as feed additive on nutrient utilization and growth in crossbred heifers fed paddy straw based ration" INDIAN JOURNAL OF ANIMAL SCIENCES, vol. 75, no. 1, January 2005 (2005-01), pages 52-55, XP008052922

## Description

### Field of the invention

The present invention relates to the use of an additive containing one or more components from plant materials, or plant extracts in order to improve the energy or nitrogen retention of the rumen fermentation, or to decrease lactic acidosis. The compounds are suitably orally administered to the ruminant via the animal feed or the drinking water.

### Background of the invention

The rumen is the first stomach of ruminants such as cattle, sheep and goats. Its structure and nutritional significance is described by Hungate (1966). The rumen enables these animals to digest high-fibre plant materials unsuitable for most non-ruminant animals. The rumen is a huge organ, containing in excess of 150 kg of digesta in dairy cows, in which all of the digestion is carried out by micro-organisms. The rumen evolved in the way it has done for the animal to benefit from the fibre-digesting activities of the microbes. The end-products, the volatile fatty acids, are absorbed across the rumen wall and used for energy and protein synthesis. The principal benefit of the rumen foregut fermentation in comparison with hindgut fermentation is that the microbial cells formed during the fermentation pass to the abomasum, where they are digested and their amino acids are absorbed. Unlike the hindgut fermentation, therefore, microbial amino acids become available to the host animal. The rumen is therefore a highly efficient organ in the context of the evolution of a herbivore subsisting on poor pasture.

Rumen fermentation also brings some disadvantages. Methane is produced as a natural consequence of the anaerobic fermentation (Hungate, 1966). The release of methane also represents a loss of energy from the animal. Furthermore, methane is a potent greenhouse gas, so in this sense, ruminants damage the environment (Leng, 1992). The ruminant is also less efficient than other species in the utilization of dietary protein. Nitrogen losses from ruminants are exceptionally high, particularly in grazing animals (Nolan, 1975). This is an environmental problem as well as an economic one, because of the impact of nitrogen-rich excreta on the environment. Furthermore, there are digestive disorders unique to ruminants which occur as a direct consequence of rumen microbial fermentation being impaired.

Chemical and antibiotic feed additives have in the past been used to alleviate some of these problems. The presence of such materials in the food chain is becoming ever less acceptable to regulatory authorities and the consumer. Thus, solutions to the problems of ruminant livestock production must be natural and sustainable.

Dietary protein entering the rumen is broken down in an apparently uncontrolled way, resulting in ammonia formation and subsequent loss of nitrogen in the urine (Nolan, 1975). The low efficiency of nitrogen retention, which represents a major economic loss, causes metabolic stress in the animal, and also places a burden on the environment, by way of nitrogen-rich wastes. If the breakdown process could be decreased these problems would be decreased.

Further, the second possibility which exists for improving the protein retention in the rumen is the suppression of the population of rumen ciliate protozoa. Protozoa consume large quantities of bacteria in the rumen, their breakdown can result in a decrease of the net yield of microbial protein from rumen fermentation of up to 50% (Wallace et al., 1997). If the protozoa could be suppressed, there would be less ammonia formation and less need for dietary protein supplementation.

Lactic acid is normally only a minor product of rumen fermentation. However, when a rapidly degraded feed is introduced too quickly, or when concentrates form a high proportion of the diet, volatile fatty acid production will exceed the buffering capacity of the rumen. This may lead to a so low pH value in the rumen that only lactic acid-producing bacteria can grow (Russell & Hino, 1985).

As lactic acid is a stronger acid than the volatile fatty acids, pH falls even more, the recovery of the normal fermentation becomes impossible, and the animal dies. More typically, however, ruminants on high-concentrate diets suffer sub-clinical acidosis, which is distressing and decreases production efficiency although not life-threatening. Chemical buffers can reverse this condition, but it would be better if the growth of the lactic acid-producing bacteria could be suppressed.

The publication WO 03/094628 describes an attempt to reduce the methane production emanating from the digestive activities of ruminants by the additions of one or more essential oil compounds selected from the group consisting of limonene, eugenol, a salicylate, quinoline, vanilla, thymol and a cresol. At the same time the microbial activities in the rumen and the production of the volatile fatty acids, e g propionic acid, can be maintained on an acceptable level.

### Objects and summary of the invention

The object of the present invention is to generally and collectively address the digestive disorders in the rumen and to promote the utilisation of energy and nitrogen sources available for the growth of the ruminant. This object can be achieved by favouring the formation of volatile fatty acids, which are absorbed across the rumen wall and are used for energy and protein synthesis, and by diminishing the breakdown of dietary protein and microbial protein. Other methods are to limit the methanogenesis and to control or suppress the lactic acid-producing bacteria.

According to the invention said object can be achieved by the use of, an additive containing one or more components of plant materials selected from the group consisting of Lonicera japonica, Gentiana asclepidea, Gentiana lutea, Eugenia caryophyllata, Bellis perennis, Olea europaea, Symphytum officinale, Carduus pycnocephalus, Paeoniae alba radix, Populus tremula, Prunus avium, Salix caprea, Rheum nobile, Helianthemum canum, Arctostaphylos uva-ursi, Peltiphyllum peltatum, Epilobium montanum, Knautia arvensis, Latuca sativa and Urtica dioica and extracts thereof, and β-myrcene. The total amount of the components to be administered to an animal is suitably from 0.02 mg to 20 g per day and kg bodyweight.

The components have all been shown to essentially contribute to the efficiency of the fermentation in the rumen without having any considerable drawbacks. Some of the components suppress the activity of protozoa and others decrease the proteolytic activity, the methanogenic activity or the production of lactic acid.

### Detailed description of the invention

Since the components have influence on the fermentation in different ways, it is advantageous to combine specific compounds to optimize the fermentation in accordance with the object of the invention.

Extensive studies have shown that the components of a subgroup consisting of plant materials from Helianthemum canum, Arctostaphylos uva-ursi, Epilobium montanum, Knautia arvensis and Peltiphyllum peltatum and extracts thereof, considerably reduce the ruminal proteolysis. In addition, all of these components inhibit the activity of protozoa and most of them have also beneficial influences on other essential fermentation parameters, such as the methane production, the amount of volatile fatty acids, the digestibility, the microbial biomass and/or the fermentation efficiency. The most suitable components are plant materials from Arctostaphylos uva-ursi, Knautia arvensis and Helianthemum canum and extracts thereof. Most preferred components are plant materials from Knautia arvensis and its extracts, especially methanol extracts.

Plant materials from Lonicera japonica, Gentiana asclepidea, Gentiana lutea, Eugenia caryophyllata, Bellis perennis, Olea europaea and Symphytum officinale and extracts thereof and β-myrcene, form another subgroup, components of which suppress the bacteriolytic activity of ciliate protozoa in the rumen fluid. Eugenia caryophyllata also increases the microbial biomass and reduces the methane production. Bellis perennis has a small reduction of the methane production and also improves the fermentation efficiency and the microbial biomass production, while Symphytum officinale increases the fermentation efficiency and reduces the acidosis effects. β-Myrcene has a moderate inhibiting effect on the protozoal activity but finds also a use for the decrease of the methane production. An extract prepared from the flowers of Lonicera japonica abolished the bacterolytic activity of ciliate protozoa. A seed preparation of Lonicera japonica also depressed the protozoal activity, but only to about 60%. Preferred components from this subgroup are plant materials from Bellis perennis, Eugenia caryophyllata, Olea europaea and Symphytum officinale and the extracts thereof, especially methanol and water extracts of Bellis perennis and Gentiana asclepidea.

Plant materials from Carduus pycnocephalus, Paeoniae alba radix, Populus tremula, Prunus avium, Salix caprea, and Rheum nobile and extracts thereof form a third subgroup, com ponents of which are suitably used to decrease the methanogenic activity in the rumen digesta. For these components no important detrimental effects have been observed on other fermentation parameters. Preferred components are the plant materials from Carduus pycnocephalus, Populus tremula, and Rheum nobile and extracts thereof, especially preferred are Carduus pycnocephalus and Rheum nobile.

Finally, a fourth subgroup is formed by plant materials from Latuca sativa and Urtica dioica and extracts thereof. These components lower the activity and/or the formation of lactic acid. Latuca sativa, sometimes called Latuca virosa, also diminishes the methane production and the protozoal activities and increases the formation of volatile fatty acids. Urtica dioica has also additional effects and inhibits the proteolysis and protozoal activities and favours the formation of volatile fatty acids.

In a suitable embodiment of the invention, the additive contains components from more than one of the subgroups defined above and it may be favourable that the additive contains components from all four subgroups. Advantageously the additive contains at least one component selected from each of the groups consisting of
i) plant materials from Knautia arvensis, Arctostaphylos uva-ursi and Helianthemum canum and extracts thereof,
ii) plant materials from Bellis perennis, Eugenia caryophyllata, Olea europaea, Lonicera japonica and Symphytum officinale and extracts thereof,
iii) plant materials from Carduus pycnocephalus, Populus tremula, and Rheum nobile and the extracts thereof, and
iv) plant materials from Latuca sativa and Urtica dioica and extracts thereof. The total amount of the components belonging to the groups i)-iv) is suitably from 20-100% by weight, preferably 40-100% by weight of the total amount of the components present according to the invention.

Extracts of plant materials according to the invention include, but are not limited to concretes, essential oils, resinoids, tinctures, absolutes and absolute oils. When producing extracts of plants or plant parts, water, organic solvents and mixtures thereof can be used in accordance with conventional methods but also dry distillation may be applied. Examples of suitable organic solvents are methanol, ethanol, propanol, butanol, ethyl acetate, methyl ethyl ether, diethyl ethers, methylene chloride, chloroform, carbon tetrachloride, benzene, toluene, petroleum ether and acetone.

The total amount of the components administered to the animal depends on whether the components are an essential oil compound, extracts of plant material or plant material. In the case that the components are all β-myrcene, and/or extracts, then the administered amount is normally from
0.1-50 mg per day and kg bodyweight, while when the components only consist of plant material, the amount administered is normally from 0.02-10 g per day and kg bodyweight.

The additive may contain other ingredients than the components of the invention. Suitably the additive contain 0.1-100%, preferably 0.2-90% by weight of the components, as well as growth improving agents, flavourings, absorbing supports and/or other feed ingredients. Preferably the total amount of the growth improving agents, the flavourings, the absorbing supports and/or the other feed ingredients is from 10 to 75% by weight of the additive. In case any of the components are plant material they are suitably dried and ground to a particulate or powder before they are mixed with the other ingredients in the additive, if any.

Example of suitable growth-improving additives and flavourings are cresol, anethol, deca-, undeca- and/or dodeca-lactones, ionones, irone, gingerol, piperidine, propylidene phatalide, butylidene phatalide, capsaicin and/or tannin. The support may contain, for example, 40-50% by weight of wood fibres, 8-10% by weight of stearine, 4-5% by weight of curcuma powder, 4-5% by weight of rosemary powder, 22-28% by weight of limestone, 1-3% by weight of a gum, such as gum arabic, 5-50% by weight of sugar and/or starch and 5-15% by weight of water. The other feed ingredients are suitably selected from the group consisting of vitamins, enzymes, mineral salts, ground cereals, protein-containing components, carbohydrate-containing components, wheat middlings and/or brans. The additive is suitably added to the feed composition according to the invention in such quantities that the feed will normally contain 0.4 ppm-80% by weight of the components.

The feed composition usually contains, calculated on the dry weight of the feed, the following ingredients.
a) 0-80% by dry weight of cereals,
b) 0-30% by dry weight of fat,
c) 0-85% by dry weight of protein-containing nutritious substances of a type other than cereals, and
d) 10 ppm - 40% by dry weight of the components of the invention.
The total amounts of a)-d) are preferably at least 50% by dry weight.

When preparing the feed composition, the additive can be mixed with the dry ingredients consisting of cereals, such as ground or crushed wheat, oats, barley, maize and rice; vegetable protein sources based on e.g. rape-seed, soya bean and sunflower seed; animal protein sources; molasses; and milk products, such as various milk powders and whey powders. After mixing all the dry ingredients, the liquid ingredients and ingredients, which after heating become liquid, can be added. The liquid ingredients may consist of lipids, such as fat, for example vegetable fat, optionally liquefied by heating, and/or of carboxylic acids, such as a fatty acid. After thorough mixing, a mealy or particulate consistency is obtained, depending on the degree grinding of the ingredients. To prevent separation during storage, water should preferably be added to the animal feed, which then is subjected to a conventional pelletizing, expanding or extruding process. Any excess water can be removed by drying. If desired, the resulting granular animal feed can also be crushed to a smaller particle size. The described feed composition is usually administered in combination with dried green forage and/or silage.

The drinking water supplement may contain at least 1%, suitably 1-99% by dry weight, preferably 10-50% by dry weight, of the components. Beside one or more of the components, the supplement may also contain 1-99% by dry weight of a large number of other ingredients. Suitable examples of other ingredients are mineral salts, vitamins, health and growth enhancing additives, flavourings, water-soluble or water-dispersible carriers, such as sugars, powdered milk, milk-by-products and cellulose derivatives, dispersing agents and stabilizers, such as water-soluble or water-dispersible polymers, and mixtures thereof. When preparing the drinking water, the supplement is normally added to the water in such an amount that the concentration of the components becomes 1 ppm-10% by weight.

Within the scope of the invention, it is also possible to produce a suspension of the feed composition. This is especially convenient if the feed is prepared for immediate consumption.

The present invention will now be further illustrated by the following Examples.

### Examples

In the present Examples a number of tests were performed with samples of plant materials, extracts of plant materials and an essential-oil compound. The samples of plant materials were freeze-dried and ground and passed through a 1 mm screen. The samples were as follows.

| Sample No. | Botanic name | Description of the sample |
|---|---|---|
| 1 | Arctostaphylos uva-ursi | Leaves and stem |
| 2 | Bellis perennis | Plant, mainly leaves |
| 3 | Carduus pycnocephalus | Mixture of stems, leaves and flowers |
| 4 | Epilobium montanum | Foliage |
| 5 | Eugenia caryophyllata | Dried embro seed |
| 6 | Gentiana asclepidea | Preparation of leaf and stem |
| 7 | Helianthemum canum | Leaves and flowers |
| 8 | Knautia arvensis | Field scabious, all overground |
| 9 | Latuca sativa | Garden lettuce, whole overground |
| 10 | Lonicera japonica | Leaves, stems and flowers |
| 11 | Lonicera japonica (flower) | Extract of flowers |
| 12 | Lonicera japonica + Magnolia officinalis | Dried leaves and flowers |
| 13 | β-Myrcene | Essential oil compound |
| 14 | Olea europaea | Dried leaves |
| 15 | Paeoniae alba radix | Root |
| 16 | Peltiphyllum peltatum | Whole overground |
| 17 | Populus tremula | Preparation of leaves and stem |
| 18 | Prunus avium | Mainly leaves and small stems |
| 19 | Rheum nobile | Leaves and stem |
| 20 | Salix caprea | Mainly leaves and small stems |
| 21 | Symphytum officinale | All over ground plant |
| 22 | Urtica dioica | Stinging nettles |

The effects on the ruminal digesta of the samples 1-22 according to the present invention were tested with regard to the bacterolytic activity of ruminal ciliated protozoa, proteolytic activity of ruminal digesta, the methane formation caused by ruminal digest, the lactic acidosis and the formation of volatile fatty acids as well as other effects on fermentation. In the tests the following methodologies were applied.

### Bacteriolytic activity of ruminal ciliate protozoa

The rate of breakdown of [¹⁴C]leucine-labelled Selenomonas ruminantium was determined by incubating strained ruminal fluid in vitro with labelled S. ruminantium in the presence of 5 mM unlabelled L-leucine as described previously (Wallace & McPherson, 1987). Under these conditions, the great majority of bacterial protein breakdown is caused by the ingestion and digestion of bacteria by ciliate protozoa. The samples were, unless stated otherwise, added at a concentration of 5 mg/ml. The sheep received a mixed grass hay:concentrate diet (Frumholtz et al., 1989).

Persistency of antiprotozoal activity The samples were subjected to a pre-incubation with Coleman's buffer, ruminal fluid or water for 24 h, then were subsequently incubated with fresh ruminal fluid in the bacteriolysis assay described above.

### Proteolytic activity of ruminal digesta

Method 1. Strained ruminal fluid from the same sheep was used to determine proteolytic activity using the ¹⁴C-casein method of Wallace (1983). Samples were added to a final concentration of 2.5 mg/ml, and an incubation time of 1 h was used.

Method 2. Based on Hoffman et al. (2003a,b). Five fistulated lactating Holstein cows, fed a typical dairy cow diet, served as donor animals. Rumen fluid was withdrawn manually from the feed mat prior to morning feeding. The rumen fluid was filtered through a 100 µm nylon net and mixed with incubation buffer ad 10% (v/v). Buffered rumen fluid in a volume of 75 ml was dispensed into the (RPT) incubation bottles containing a substrate mixture of 450 mg maize silage, 225 mg barley grain (both ground to pass a 1 mm screen), and a protein supplement composed of 150 mg defatted, raw soybean meal and 10 mg bovine serum albumin. This substrate without addition of test plants was incubated as negative control. When test plants were added, they replaced the silage. Monensin used as a positive control was dissolved in ethanol (14 mg/ml) and 11.25 µl of this stock solution were dosed into each flask immediately after filling to an end concentration of 3 µM monensin. All flasks were incubated at 39°C for up to 12 h. Two replicates of each treatment were sampled at regular intervals while a third subreplicate was reserved for gas reading. At each sampling time 2 x 900 µl were withdrawn from the incubation bottle under vigorous stirring and prepared for the determination of SCFA, ammonia and protein concentration. SDS-PAGE was carried out by the Laemmli method, total protein quantitation by dot blot according to Hoffman et al. (2002).

### General effects on ruminal fermentation

### Hohenheim Methods for general effects on fermentation

### Experimental design:

Incubation system: Reading Pressure Technique (RPT), 75 ml, total running time 24 h
Donor animals: 3 non-lactating Holstein cows
Basal substrate: Grass silage 0.750 g (control)
Inclusion level of test plants: 10% (silage replaced)
Gas readings: 2, 4, 6, 8, 10, 12, 16, 24 h
Sampling: 24 h for SCFA and TD (NDS treatment in nylon bags)

6, 8, 10 h for SCFA (900 µl) and RNA (300 µl, maxima analyzed) Parallels: 3 independent runs (3 different donor animals) in duplicates

### RPT buffer

| | Components | Molarity | final conc. |
|---|---|---|---|
| | Ammonium bicarbonate | 60.1 | 13.5 mM |
| | Natrium bicarbonate | 84.0 | 86.5 mM |
| | diNatrium Hydrogen Phosphate | 142.0 | 5.5 mM |
| | Postassium diHydrogen Phosphate | 136.1 | 9.5 mM |
| | Magnesium Sulfate 7xH₂O | 246.5 | 0.5 mM |
| | Microminerals | | 0.020% |
| 1% Reszurine | | | 0.001% |
| | d H₂O | | |
| | Reducing solution | | 6% |
| | Rumen fluid | | 10% |
| | total volume | | |
| Reducing solution | | | |

| | Components | MW | final conc. |
|---|---|---|---|
| | Cysteine HCl | 52.9 | 0.118 M |
| 1M NaOH | | 40.0 | 0.040 % |
| | Na₂S | 240.2 | 0.026 N |
| | total volume | make up to with dH2O | |
| 0.5x Microminerals | | | |

| | Components | MW | final conc. |
|---|---|---|---|
| | Calcium Cloride 2x H₂O | 147.0 | 0.45 M |
| | Mangan Chloride 4xH₂O | 197.9 | 0.25 M |
| | Cobald Chloride 6xH₂O | 237.9 | 0.02 M |
| | Ferric Trichloride 6xH₂O | 270.3 | 0.15 M |
| | total volume | make up to with d H₂O | |

### Detailed description of methods:

### In vitro incubation

The rumen fluid was collected from cannulated cows receiving a grass silage hay mix at maintenance fed in two equal portions at 8:00 and 16:00. Rumen fluid was collected before feeding in thermos bottles, filtered through a 100 µm nylon net and added to the reduced buffer-mineral solution. All steps were carried out at 39°C under CO₂ to maintain anaerobic conditions.

Substrates were incubated at a ratio of 10mg/ml medium, i.e. 750 mg of substrate was incubated with 75 ml of buffered rumen fluid containing 7.5 ml of filtered rumen fluid (10% v/v rumen fluid).

Gas volume or the pressure was recorded frequently at 2, 4, 6, 8, 10, 12, 16, and 24 hours of incubation and samples were taken after 6, 8, 10, and 24 hours.

### Analysis

Gas pressure is recorded in the serum bottles of the RPT system and converted into volume using a experimentally determined calibration curve. After each measurement the gas is released from the serum bottles. *In vitro* true digestibility was determined by transferring the incubation content to preweighed polyester bags (Ankom 51 µm pore size). The bags were heat-sealed, cooked in neutral detergent solution for 1 hour, dried at 105°C over night and weighted for determination of digestibility. SCFAs were determined by gas chromatography using a stainless steel column packed with GP 10% SP 1000 1% H₃PO₄, Chromosob W AW (Suppelco Inc. Bellafonte, PA). To 0.9 ml of the incubation supernatant 0.1 ml formic acid containing the internal standard (1% Methylbutryric acid) was added. Proteins were precipitated over night at 4°C. Samples were centrifuged (30,000g, 10 min, 4°C) and the supernatant was collected into appropriate GC-vials for analysis.

RNA was extracted according to a modified phenol chloroform protocol. 600µl pH 5.1 phenol, 270µl pH 5.1 buffer, 30µl SDS (20% w/v) and 1.0 g of zirconium beads were added to the samples (300µl). Cells were lysed by beating the samples for 2 x 2 minutes in the bead-beater (50 Hz) interrupted by a 10 min incubation at 60°C. Samples were cooled on ice for 10 min and 300µl chloroform were added. After shaking vigorously and another 10 min at RT samples were centrifuged (10,000g, 5 min, 4°C) to separate aqueous and organic phases. Aqueous phase was removed quantitatively and transferred to a vial containing 300µl NH₄-acetate (7.5M) and 900µl isopropanol. Samples were incubated over night at -20°C and nucleic acids were precipitated by centrifugation (16,000g, 10 min, 4°C). Supernatant was discarded and the samples were washed once in 80% ethanol. Nucleic acids were loaded on agarose gels and quantified densitometrically after staining with ethidium bromide against a calibration curve (25 -300 ng/µl).

### Reading Methods for general effects on fermentation

A series of thirteen consecutive fermentations, one per week, was undertaken using the Reading Pressure Technique [RPT], to identify potential effects from including these test materials on the fermentation and degradation characteristics of a basal forage [maize silage]. All materials were examined in triplicate and at two inclusion levels [100 and 400 mg g⁻¹ DM substrate], with the exception of β-myrcene [10 and 40 mg g⁻¹, inclusion levels]. The maize silage was pre-dried and milled to pass a 2 mm screen. A total of 1.0 g blended substrate [silage plus substrate] was placed in each fermentation flask. Buffered incubation medium [90 ml] was then added, the flasks sealed and stored overnight at room temperature. Prior to inoculation with prepared rumen fluid, flask contents were raised to 39°C. Rumen fluid was obtained manually [hand-squeezed contents] prior to feeding [07.00h] from two lactating dairy cows offered a maize/grass silage : concentrate [60:40] ration *ad libitum.* The fluid was filtered through two layers of muslin and held under CO₂ at 39°C until use. Ten ml prepared fluid was added to each flask and these incubated at 39°C for the duration of the study. Six negative controls [buffered medium plus rumen fluid only] were included in each run to correct gas values and degradation residues for direct rumen fluid effects. In addition un-supplemented maize silage [1.0 g flask⁻¹] was included in all runs [six flasks per run]. Results within studies were expressed relative to the positive control values.

Headspace gas pressure readings were obtained 2, 4, 6, 8, 10, 12, 14, 16, 18, 21 and 24 hours post-inoculation. Following the last measurement approximately 3.0 ml fermentation medium was sampled from each flask and bulked by test material / inclusion level and stored frozen [-20°C] until later analysis for VFA composition using a Varian 3600 GC. Fermentation residues were recovered by filtering flask contents through 60 ml Gooch crucibles [porosity 1,100 to 160 µm] under light vacuum. The residues were then dried [100°C for 24 hours], weighed, ashed [500°C overnight] and reweighed. The quantity of dry matter [DM] and organic matter [OM] determined were used to assess iDMD and iOMD [*in vitro* DM and OM degradation, respectively]. The extent [ml gas g⁻¹ OM incubated] and rate of fermentation gas release [ml h⁻¹] were generated using a previously derived quadratic function to convert pressure to volume. Feed fermentation efficiency [FE] was estimated as iDMD [g kg⁻¹] / cumulative gas [ml] release at 24 h post-inoculation. Statistical differences between treatment means and the positive control [maize silage alone] were assessed using Students T-test and significance declared at P>0.05.

### León Methods for general effects on fermentation

Rumen cannulated sheep fed on a diet consisting of 75% alfalfa hay and 25% barley (free access to vitamin-mineral supplements and water) were used as donors for rumen fluid. Rumen fluid was collected just before the morning meal.

The substrate used for the batch cultures consisted of 50% alfalfa hay, 40% grass hay and 10% barley grain, milled in a hammer mill with a 1-mm screen. The amount of substrate used was 500 mg, and inclusion rate of the test plant was 10% (ca. 50 mg). Both were weighed into serum bottles, where 50 ml of buffered rumen fluid (containing 10 ml of filtered rumen fluid and 40 ml of the medium described by Goering & Van Soest, 1970) were dispensed an-aerobically. The bottles were sealed and incubated at 39°C. After 24 h of incubation, the ensuing measurements were recorded:
- Total gas production (ml), using a pressure transducer and collecting the gas in a calibrated syringe.
- pH in the incubation medium.
- Volatile fatty acids concentration in the incubation medium, by GC (VFA was also measured at inoculation time to calculate VFA production after 24 h).
- DM incubation residue, by filtering the bottle contents in sintered crucibles and weighing the residue after drying. Thereafter, the neutral detergent fibre (NDF) content of the residue was determined to calculate the amount of un-degraded NDF. From this, DM and NDF disappearance were estimated.

Three replicates per plant were incubated, and blanks (no substrate no plant) and controls (500 mg substrate without any plant, and 550 mg substrate without any plant) were used.

### Methane formation by ruminal digesta

Incubations carried out by the León general method were analyzed for methane formation. A representative sample of the gas produced was taken for subsequent analysis. Sampling was conducted using special gas-tight glass syringes fitted with a valve allowing for sample locking and storage. The sample was collected directly from the headspace, after inserting the needle through the se ptum of the stopper, taking a sample in the syringe and closing the valve. This sample was collected after measuring total gas production, assuming that gas composition was the same in the gas released and in that remaining in the headspace.

Methane concentration in the gas produced was determined by gas chromatography. The gas sample (300 to 500 µl) was injected directly from the gas-tight syringe to the corresponding port. The GC parameter settings used for the analysis were:
Instrument: Shimadzu GC-14B provided with a flame ionisation detector (FID)
Column: 2.3 meters length x 2.1 mm internal diameter stainless steel column packed with 60/80 mesh Carboxen 1000 stationary phase
Carrier gas: Helium, flow rate (100 kPa), Constant flow mode
Temperatures:
Detector: FID. Temperature 200°C, Synthetic Air flow rate (50 kPa), H₂ flow rate (50 kPa) Injector: Temperature 200°C
Column-oven: 170°C (isothermal)

The standard used for calibration was pure methane (99.9%). The standard curve was established by plotting a linear regression of the standard quantities injected of methane versus the area of the peaks to obtain the response factor.

### Acidosis assay

A model was used in which the decrease in incubation medium pH and the concentration of lactic acid produced, relative to the controls over a 48-h fermentation period provided an indication of the ability of the test substrates to offset potential acidotic effects. In this study milled wheat grain [2 mm] was used as the basal substrate and the test materials included at 100 mg g⁻¹ DM [or 10 mg g⁻¹ DM β-myrcene]. As before 1.0 g total substrate was added to each flask. To enhance lactic acid production, the buffer solution prepared was diluted by 50 percent. This was to ensure that the buffering capacity of the incubation medium was exceeded and the resulting decrease in pH encouraged growth of Lactobacilli and other lactic acid producing bacteria. Rumen fluid was obtained and prepared as previously described except that the two cows used were offered an early lactation ration with a high level of coarse milled wheat. Fluid pH measurements were made one hour after inoculation [start pH] then after a further 23 and 47 hours, by inserting a pH electrode directly into each flask. Immediately following the last measurement approximately 3.0 ml fluid were taken from each flask, bulked by sample and stored frozen at-20°C until analyzed enzymatically for L [+] lactic acid.

Results are presented as absolute differences relative to the controls. A positive effect of on acidosis was identified as reduced rate of pH decline to 24 h, a higher endpoint pH and a lowered lactic acid concentration.

### Results

The results obtained from the measurements of the fermentation activities are shown in Tables 2a, 2b and 2c below.

The results obtained in the protozoal assay, the proteolytic assay by method 1, and in methane formation are shown in the same tables.

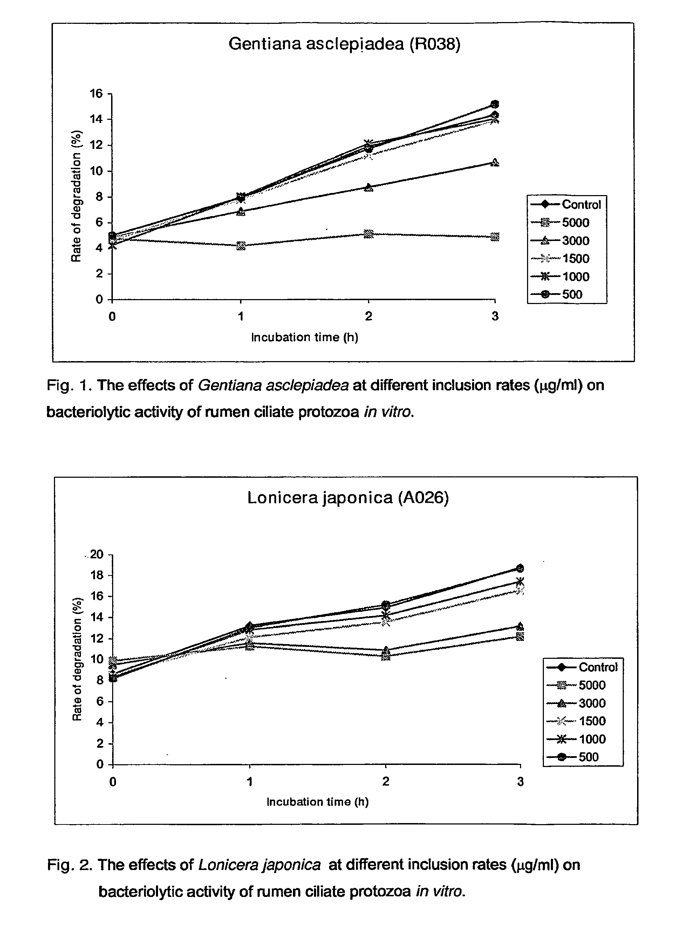

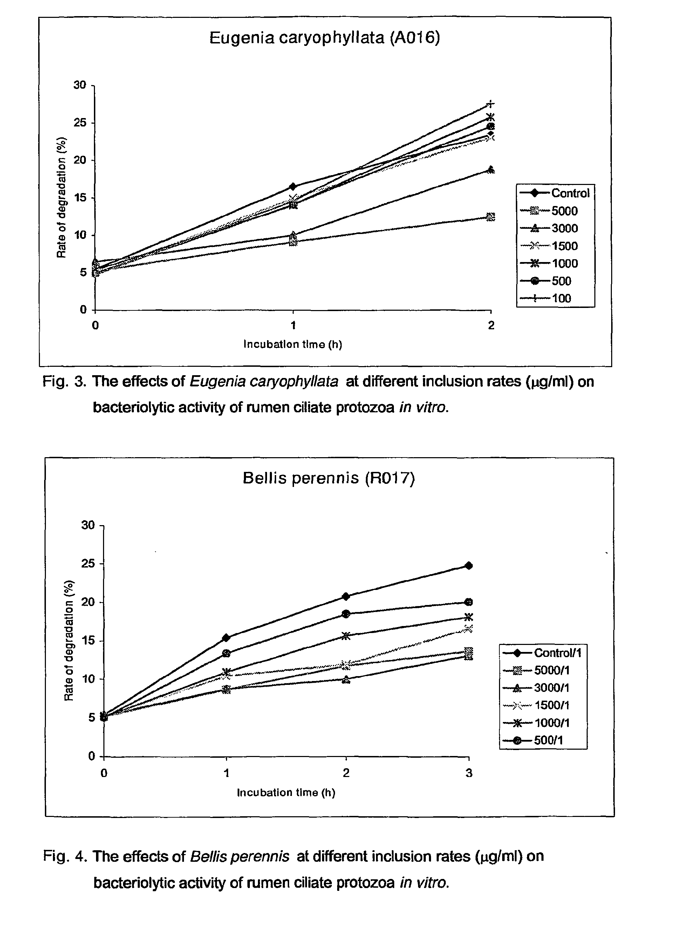

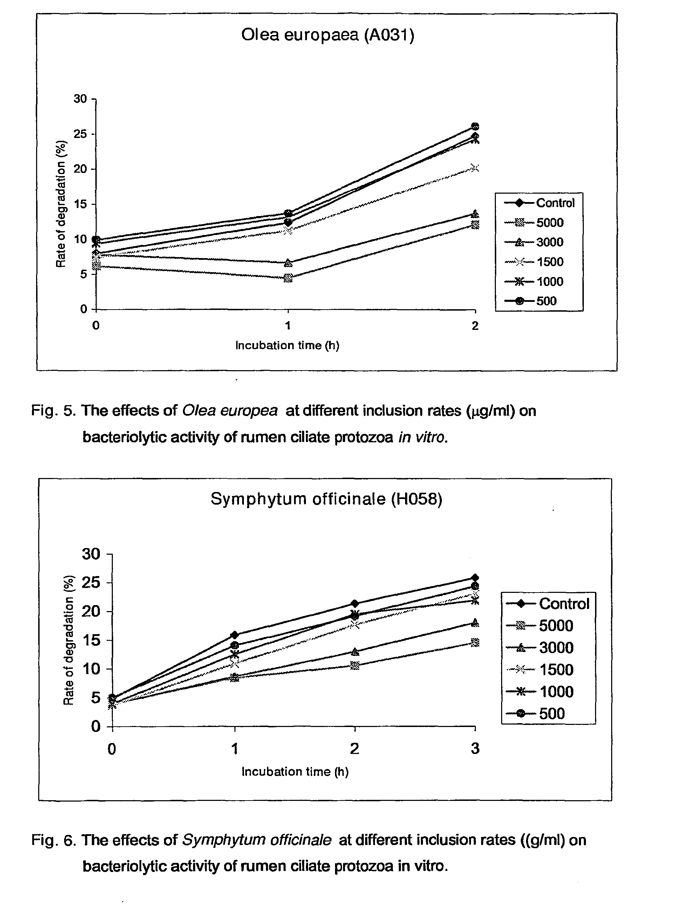

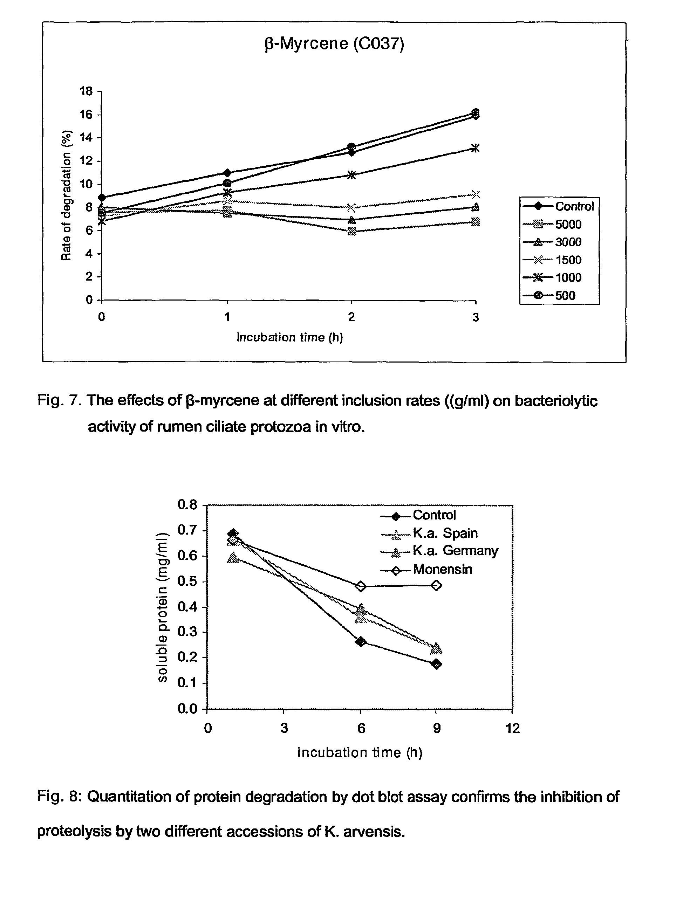

The effects of selected plant/plant extracts at different inclusion rates on bacteriolytic activity of the protozoa in vitro are shown in Fig. 1 to Fig. 7. Persistency of the bacteriolytic activity of the protozoa was higher in ruminal fluid than in buffer or water, particularly with *G. asclepiadea* (Table 1). The influence of K. arvensis is shown in Fig. 8.

**Table 1. Influence of pre-incubation in ruminal fluid or Coleman's buffer on the ability of samples to inhibit the bacteriolytic activity of ruminal ciliate protozoa**

| Bacteriotytic activity (% of activity in control with no addition or pre-incubation) | *Bellis perennis* | *Gentiana asclepiadea* |
|---|---|---|
| Control, no addition | 100 | 100 |
| + additive, no preincubation | 0 | 42 |
| + additive, 24h preincubation in RF | 0 | 20 |
| + additive, 24h preincubation in Coleman's buffer | 13 | 28 |
| + additive, 24h preincubation in water | 24 | 68 |

**Table 2a. Effects on selected plant materials, plant extracts or nature-identical components on fermentation. The values in absence of plant material are 100.**

| *General effects on fermentation* | | Sample No. | | | | | | | |
|---|---|---|---|---|---|---|---|---|---|
| | Amount in feed % . | 1 | 2 | 3 | 4 | 5 | 6 | 7 | 8 |
| Reading diet | | | | | | | | | |
| Dry matter digestion | 10 | 95 | 97 | 99 | 93 | | 102 | 85 | 99 |
| | 40 | 82 | 106 | 99 | 84 | | 98 | 43 | 96 |
| Cumulative gas, 24 h | 10 | 86 | 97 | 92 | 85 | | 96 | 79 | 96 |
| | 40 | 72 | 88 | 88 | 68 | | 92 | 42 | 90 |
| Fermentation efficiency | 10 | 110 | 100 | 108 | 110 | | 106 | 107 | 103 |
| | 40 | 114 | 121 | 112 | 124 | | 106 | 101 | 107 |
| Hohenheim diet | | | | | | | | | |
| Digestibility | 10 | 102 | 102 | 103 | 103 | 103 | 101 | 103 | 102 |
| Gas production | 10 | 105 | 102 | 108 | 108 | 103 | 104 | 108 | 99 |
| Fermentation efficiency | 10 | 97 | 100 | 95 | 95 | 99 | 97 | 95 | 102 |
| SCFA | 10 | 110 | 118 | 124 | 124 | 106 | 111 | 119 | 96 |
| C3/C2 | 10 | 106 | 102 | 108 | 108 | 95 | 97 | 107 | 96 |
| Leon diet | | | | | | | | | |
| Digestibility | 10 | 104 | 105 | 106 | 106 | 97 | 102 | 103 | 105 |
| App. digestibility | 10 | 95 | 98 | 97-107 | 101 | 94 | 98 | 96 | 101 |
| Gas production | 10 | 105 | 103 | 104 | 104 | 102 | 104 | 103 | 96 |
| Fermentation efficiency | 10 | 100 | 102 | 102 | 102 | 96 | 98 | 101 | 115 |
| Microbial biomass | 10 | 104 | 108 | 110 | 110 | 98 | 97 | 105 | 125 |
| C3/C2 | 10 | 101 | 100 | 95 | 95 | 102 | 100 | 99 | 96 |
| TVFA | 10 | 108 | 102 | 106 | 106 | 90 | 97 | 104 | 108 |

| *Specific effects on fermentation* | | | | | | | | | |
|---|---|---|---|---|---|---|---|---|---|
| Protozoal activity | 5mg/ml | 87 | 7 | 84 | 70 | 2 | 4 | 73 | 82 |
| Methane formation, Leon | 10% of feed | 83 | 94 | 70-92 | 102 | 93 | 106 | 90 | 97 |
| Proteolysis, Method 1 | | 79 | 90 | 87 | 76 | 102 | 104 | 78 | 105 |
| Acidity, 24 h 48 h | | 99 | 77 | 76 | 88 | 61 | 79 | 80 | 89 |
| | | 100 | 77 | 71 | 100 | 51 | 82 | 70 | 84 |

**Table 2b. Effects on selected plant materials, plant extracts or nature-idantical components on fermentation. The values in absence of plant material are 100.**

| *General effects on fermentation* | | Sample No. | | | | | | | |
|---|---|---|---|---|---|---|---|---|---|
| | Amount in feed % | 9 | 10 | 11 | 12 | 13 | 14 | 15 | 16 |
| Reading diet | | | | | | | | | |
| Dry matter Digestion | 10 | 102 | 99 | 98 | 97 | 103 | 92 | 104 | 90 |
| | 40 | 109 | 106 | 98 | 89 | 89 | 89 | 112 | 49 |
| Cumulative gas, 24 h | 10 | 95 | 95 | 91 | 90 | 100 | 84 | 100 | 88 |
| | 40 | 79 | 91 | 82 | 82 | 82 | 63 | 107 | 51 |
| Fermentation Efficiency | 10 | 107 | 104 | 108 | 107 | 103 | 110 | 104 | 102 |
| | 40 | 139 | 116 | 119 | 109 | 109 | 141 | 104 | 96 |
| Hohenheim diet | | | | | | | | | |
| Digestibility | 10 | 102 | 100 | 97 | 99 | 100 | 101 | 99 | 102 |
| Gas production | 10 | 100 | 98 | 99 | 106 | 101 | 111 | 104 | 103 |
| Fermentation Efficiency | 10 | 102 | 102 | 98 | 94 | 99 | 91 | 94 | 99 |
| SCFA | 10 | 98 | 105 | 97 | 103 | 103 | 110 | 105 | 118 |
| C3/C2 | 10 | 98 | 98 | 97 | 98 | 104 | 103 | 100 | 102 |
| Leon diet | | | | | | | | | |
| Digestibility | 10 | 102 | 105 | 95 | 97 | 102 | 100 | 104 | 103 |
| App. Digestibility | 10 | 96 | 100 | 98 | 97 | 93 | 93 | 110-113 | 107 |
| Gas production | 10 | 107 | 98 | 98 | 99 | 97 | 106 | 95 | 98 |
| Fermentation Efficiency | 10 | 97 | 106 | 98 | 99 | 109 | 96 | 117 | 105 |
| Microbial biomass | 10 | 102 | 112 | 93 | 94 | 115 | 95 | 116 | 110 |
| C3/C2 | 10 | 105 | 111 | 106 | 105 | 105 | 109 | 105 | 95 |
| TVFA | 10 | 105 | 94 | 99 | 103 | 96 | 108 | 120 | 97 |

| *Specific effects on fermentation* | | | | | | | | | |
|---|---|---|---|---|---|---|---|---|---|
| Protozoal activity | 5mg/ml | 67 | 40 | 18 | 5 | 68 | 12 | 103 | 83 |
| Methane Formation, Leon | 10% of feed | 86 | 108 | 106 | 108 | 90 | 100 | 46-92 | 93 |
| Proteolysis, Method 1 | | 154 | 112 | 113 | 92 | 110 | 70 | 83 | 73 |
| Acidity, 24 h 48 h | | 58 | 106 | 91 | 87 | 105 | 97 | 108 | 66 |
| | | 63 | 100 | 92 | 82 | 115 | 91 | 109 | 91 |

**Table 2c. Effects on selected plant materials, plant extracts or nature-identical components on fermentation. The values in absence of plant material are 100.**

| *General effects on fermentation* | | Sample No. | | | | | |
|---|---|---|---|---|---|---|---|
| | Amount % | 17 | 18 | 19 | 20 | 21 | 22 |
| Reading diet | | | | | | | |
| Dry matter Digestion | 10 | 96 | 99 | 97 | 97 | 93 | 99 |
| | 40 | 97 | 98 | 90 | 81 | 90 | 89 |
| Cumulative gas, 24 h | 10 | 93 | 98 | 90 | 89 | 87 | 90 |
| | 40 | 84 | 89 | 64 | 76 | 80 | 78 |
| Fermentation Efficiency | 10 | 104 | 100 | 107 | 109 | 108 | 110 |
| | 40 | 115 | 110 | 141 | 107 | 112 | 115 |
| Hohenheim diet | | | | | | | |
| Digestibility | 10 | 100 | 101 | 97 | 102 | 101 | 100 |
| Gas production | 10 | 99 | 103 | 96 | 102 | 105 | 99 |
| Fermentation Efficiency | 10 | 101 | 98 | 102 | 101 | 96 | 101 |
| SCFA | 10 | 107 | 101 | 98 | 103 | 105 | 107 |
| C3/C2 | 10 | 100 | 93 | 100 | 91 | 97 | 100 |
| Leon diet | | | | | | | |
| Digestibility | 10 | 99 | 103 | 102 | 105 | 99 | 99 |
| App. Digestibility | 10 | 97-105 | 90-106 | 89-106 | 78-102 | 78 | 95 |
| Gas production | 10 | 95 | 100 | 104 | 109 | 98 | 95 |
| Fermentation Efficiency | 10 | 104 | 104 | 99 | 96 | 100 | 104 |
| Microbial biomass | 10 | 102 | 107 | 107 | 102 | 96 | 102 |
| C3/C2 | 10 | 103 | 92 | 107 | 99 | 86 | 103 |
| TVFA | 10 | 104 | 105 | 98 | 103 | 93 | 104 |

| *Specific effects on fermentation* | | | | | | | |
|---|---|---|---|---|---|---|---|
| Protozoal activity | 5mg/ml | 84 | 83 | 57 | 76 | 13 | 83 |
| Methane formation, Leon | 10% of feed | 74-89 | 79-85 | 75-78 | 70-86 | 78 | 93 |
| Proteolysis, Method 1 | 2.5 mg/ml | 87 | 120 | 97 | 97 | 105 | 83 |
| Acidity, 24 h 48 h | | 78 | 82 | 69 | 72 | 14 | 77 |
| | | 73 | 74 | 53 | 56 | 58 | 79 |

From the results obtained it is evident that the tested components of the invention have positive effects on the rumen fermentation, which effects will support the objects of the present invention.

### Literature cited

Hoffman EM, Meutzel S, Becker K (2002), A modified dot-blot method of protein determination applied in the tannin-protein-precipitation assay to facilitate the evaluation of tannin activity in animal feeds, Br.J.Nutr. 87: 421-426
Hoffman EM, Meutzel S, Becker K (2003), Effects of Moringa oleifera seed extract on rumen fermentation in vitro, Arch.Anim.Nutr. 57: 65-81
Hoffman EM, Meutzel S, Becker K (2003), The fermentation of soybean meal by rumen microbes in vitro reveals different kinetic features for the inactivation and the degradation of trypsin inhibitor protein, Anim.Feed Sci.Technol. 106: 189-197
Hungate RE (1966), The rumen and its microbes, Academic Press, New York and London Leng RA (1992), Ruminant production and greenhouse gas emissions, Proc.NZ Soc.Anim.Prod. 52(Suppl.): 15-23
Nagaraja TG, Newbold CJ, Van Nevel CJ, Demeyer DI (1997), Manipulation of ruminal fermentation, in The rumen microbial ecosystem, ed. Hobson PN and Stewart CS, Chapman&Hall, London p 523-632
Nolan JV (1975), Quantitative models of nitrogen metabolism in sheep, in Digestion and Metabolism in the Ruminant, ed. McDonald IW and Warner ACI, University of New England Publishing Unit, Armidale, Australia p 416-431
Russell JB, Hino T (1985), Regulation of lactate production in Streptococcus bovis: a spiraling effect that contributes to rumen acidosis, J.Dairy Sci. 68: 1712-1721
Wallace RJ, McPherson CA (1987), Factors affecting the rate of breakdown of bacterial protein in rumen fluid, Br.J.Nutr. 58: 313-323
Wallace RJ (1983), Hydrolysis of 14 C-labelled proteins by rumen micro-organisms and by proteolytic enzymes prepared from rumen bacteria, Br.J.Nutr. 50: 345-355
Wallace RJ, Onodera R, Cotta MA (1997), Metabolism of nitrogen-containing compounds, in The rumen microbial ecosystem, ed. Hobson PN and Stewart CS, Chapman & Hall, London p 283-328

## Claims

1. Use of an additive containing one or more plant material(s) or plant extract(s) to affect the rumen fermentation and to increase the availability of energy and nitrogen sources for a ruminant, **characterized in that** the one or more plant material or plant extract is selected from the group consisting of Lonicera japonica, Gentiana asclepidea, Gentiana lutea, Eugenia caryophyllata, Bellis perennis, Olea europaea, Symphytum officinale, Carduus pycnocephalus, Paeoniae alba radix, Populus tremula, Prunus avium, Salix caprea, Rheum nobile, Helianthemum canum, Arctostaphylos uva- ursi, Peltiphyllum peltatum, Epilobium montanum, Knautia arvensis, Lactuca sativa and Urtica dioica and extracts thereof, and beta-myrcene.

2. Use in accordance with claim 1, **characterized in that** the components selected contain one or more components from the subgroup consisting of plant material from Helianthemum canum, Arctstaphylos uva-ursi, Epilobium montanum, Knautia arvensis and Peltiphyllum peltatum and extracts thereof, to decrease the proteolytic activity of the ruminal digestion.

3. Use in accordance with claim 1 or 2, **characterized in that** the components selected contain one or more components from the group comprising of plant materials from Lonicera japonica, Gentiana asclepidea, Eugenia caryophyllata, Bellis perennis, Olea europaea, Symphytum officinale and extracts thereof, and beta-myrcene, to suppress the activity of protozoa.

4. Use in accordance with any one of the claims 1-3, **characterized in that** the components selected contain one or more components from the groups consisting of plant materials from Carduus pycnocephalus, Paeoniae alba radix, Populus tremula, Prunus avium, Salix caprea, and Rheum nobile and extracts thereof, to decrease the methanogenic activity of the ruminal digestion.

5. Use in accordance with any one of the claims 1-4, **characterized in that** the components selected contain one or two of the components from the group consisting of plant materials from Lactuca sativa and Urtica dioica and extracts thereof, to decrease the production of lactic acid.

6. Use in accordance with the claims 2-5, **characterized in that** the additive contains components from at least two of the subgroups defined in claims 2-5.

7. Use in accordance with claim 6, **characterized in that** it contains components from all subgroups defined in claims 2-5.

8. Use in accordance with claim 7, **characterized in that** at least one of the components is selected from each of the groups i) consisting of plant materials from Knautia arvensis, Arctostaphylos uva-ursi and Helianthemum canum and extracts thereof, ii) consisting of plant materials from Bellis perennis, Eugenia caryophyllata, Olea europaea, Lonicera japonica and Symphytum officinale and extracts thereof, iii) consisting of plant materials from Carduus pycnocephalus, Populus tremula, and Rheum nobile, and extracts thereof, and iv) consisting of plant materials from Lactuca sativa and Urtica dioica and extracts thereof.

9. Use in accordance with claim 8, **characterized in that** the total amount of the components belonging to the groups i) -iv) is 20-100% by weight of the total amount of the components present in the additive.

10. Use in accordance with claims 1-7, **characterized in that** the total amount of the components to be administered is from 0.02 mg to 20 g per day and kg bodyweight of the ruminant.

## Patentansprüche

1. Verwendung eines Zusatzstoffs, der ein oder mehrere Pflanzenmaterial(ien) bzw. einen oder mehrere Pflanzenextrakt(e) enthält, zur Beeinflussung der Rumenfermentation und zum Erhöhen der Energie- und Stickstoffquellenverfügbarkeit für einen Wiederkäuer, **dadurch gekennzeichnet, dass** das eine oder die mehreren Pflanzenmaterial(ien) bzw. der eine oder die mehreren Pflanzenextrakt(e) aus der Gruppe bestehend aus Lonicera japonica, Gentiana asclepidea, Gentiana lutea, Eugenia caryophyllata, Bellis perennis, Olea europaea, Symphytum officinale, Carduus pycnocephalus, Paeoniae alba radix, Populus tremula, Prunus avium, Salix caprea, Rheum nobile, Helianthemum canum, Arctostaphylos uva-ursi, Peltiphyllum peltatum, Epilobium montanum, Knautia arvensis, Lactuca sativa und Urtica dioica und Extrakten davon sowie beta-Myrcen ausgewählt ist.

2. Verwendung nach Anspruch 1, **dadurch gekennzeichnet, dass** die ausgewählten Komponenten eine oder mehrere Komponente(n) aus der Untergruppe bestehend aus Pflanzenmaterial aus Helianthemum canum, Arctostaphylos uva-ursi, Epilobium montanum, Knautia arvensis und Peltiphyllum peltatum und Extrakten davon enthalten, zur Verringerung der proteolytischen Aktivität der Rumenverdauung.

3. Verwendung nach Anspruch 1 oder 2, **dadurch gekennzeichnet, dass** die ausgewählten Komponenten eine oder mehrere Komponenten aus der Gruppe umfassend Pflanzenmaterialien aus Lonicera japonica, Gentiana asclepidea, Eugenia caryophyllata, Bellis perennis, Olea europaea, Symphytum officinale und Extrakten davon sowie beta-Myrcen enthalten, zum Unterdrücken der Protozoenaktivität.

4. Verwendung nach einem der Ansprüche 1 bis 3, **dadurch gekennzeichnet, dass** die ausgewählten Komponenten eine oder mehrere Komponenten aus den Gruppen bestehend aus Pflanzenmaterialien aus Carduus pycnocephalus, Paeoniae alba radix, Populus tremula, Prunus avium, Salix caprea und Rheum nobile und Extrakten davon enthalten, zur Verringerung der methanogenen Aktivität der Rumenverdauung.

5. Verwendung nach einem der Ansprüche 1 bis 4, **dadurch gekennzeichnet, dass** die ausgewählten Komponenten eine oder zwei der Komponenten aus den Gruppen bestehend aus Pflanzenmaterialien aus Lactuca sativa und Urtica dioica und Extrakten davon enthalten, zur Verringerung der Milchsäureproduktion.

6. Verwendung nach den Ansprüchen 2-5, **dadurch gekennzeichnet, dass** der 2usatzstoff Komponenten aus mindestens zwei der in den Ansprüchen 2-5 definierten Untergruppen enthält.

7. Verwendung nach Anspruch 6, **dadurch gekennzeichnet, dass** sie Komponenten von allen in den Ansprüchen 2-5 definierten Untergruppen enthält.

8. Verwendung nach Anspruch 7, **dadurch gekennzeichnet, dass** mindestens eine der Komponenten aus jeder der folgenden Gruppen ausgewählt ist: i) der Gruppe, bestehend aus Pflanzenmaterial aus Knautia arvensis, Arctostaphylos uva-ursi und Helianthemum canum und Extrakten davon, ii) der Gruppe, bestehend aus Pflanzenmaterial aus Bellis perennis, Eugenia caryophyllata, Olea europaea, Lonicera japonica und Symphytum officinale und Extrakten davon, iii) der Gruppe bestehend aus Pflanzenmaterialien aus Carduus pycnocephalus, Populus tremula und Rheum nobile und Extrakten davon, sowie iv) der Gruppe bestehend aus Pflanzenmaterialien aus Lactuca sativa und Urtica dioica und Extrakten davon.

9. Verwendung nach Anspruch 8, **dadurch gekennzeichnet, dass** die Gesamtmenge der zu den Gruppen i) -iv) gehörenden Komponenten 20-100 Gew.-% der Gesamtmenge der in dem Zusatzstoff vorliegenden Komponenten ausmacht.

10. Verwendung nach den Ansprüchen 1-7, **dadurch gekennzeichnet, dass** die Gesamtmenge der zu verabreichenden Komponenten 0,02 mg bis 20 g pro Tag pro kg Körpergewicht des Wiederkäuers beträgt.

## Revendications

1. Utilisation d'un additif contenant un ou plusieurs matériaux de plantes ou extraits de plantes pour influer sur la fermentation du rumen et pour augmenter la disponibilité des sources d'énergie et d'azote pour un ruminant, **caractérisée en ce que** les un ou plusieurs matériaux de plantes ou extraits de plantes sont choisis dans le groupe constitué par *Lonicera japonica, Gentiana asclepidea, Gentiana lutea, Eugenia caryophyllata, Bellis perennis, Olea europaea, Symphytum officinale, Carduus pycnocephalus, Paeoniae alba radix, Populus tremula, Prunus avium, Salix caprea, Rheum nobile, Helianthemum canum, Arctostaphylos uva-ursi, Peltiphyllum peltatum, Epilobium montanurh, Knautia arvensis, Latuca sativa* et *Urtica dioica* et des extraits de ceux-ci, et le P-myrcène.

2. Utilisation selon la revendication 1, **caractérisée en ce que** les composants choisis contiennent un ou plusieurs composants provenant du sous-groupe constitué de matériaux de plantes issus de *Helianthemum canum, Arctostaphylos uva-ursi, Epilobium montanum, Knautia arvensis* et *Peltiphyllum peltatum,* et des extraits de ceux-ci, afin de réduire l'activité protéolytique de la digestion ruminale.

3. Utilisation selon la revendication 1 ou 2, **caractérisée en ce que** les composants choisis contiennent un ou plusieurs composants provenant du groupe constitué de matériaux de plantes issus de *Lonicera japonica, Gentiana asclepidea, Eugenia caryophyllata, Bellis perennis, Olea europaea, Symphytum officinale,* et des extraits de ceux-ci, et de bêta-myrcène, afin de supprimer l'activité des protozoaires.

4. Utilisation selon l'une quelconque des revendications 1-3, **caractérisée en ce que** les composants choisis contiennent un ou plusieurs composants provenant du groupe constitué de matériaux de plantes issus de *Carduus pycnocephalus, Paeoniae alba radix, Populus tremula, Prunus avium, Salix caprea,* et *Rheum nobile,* et des extraits de ceux-ci, afin de réduire l'activité méthanogène de la digestion ruminale.

5. Utilisation selon l'une quelconque des revendications 1-4, **caractérisée en ce que** les composants choisis contiennent un ou deux composants provenant du groupe constitué de matériaux de plantes issus de *Latuca sati va* et *Urtica dioica,* et des extraits de ceux-ci, afin de réduire la production d'acide lactique.

6. Utilisation selon les revendications 2-5, **caractérisée en ce que** l'additif contient des composants issus d'au moins deux parmi les sous-groupes définis selon les revendications 2-5.

7. Utilisation selon la revendication 6, **caractérisée en ce qu'**il contient des composants issus de tous les sous-groupes définis selon les revendications 2-5.

8. Utilisation selon la revendication 7, **caractérisée en ce qu'**au moins l'un des composants est choisi dans chacun des groupes i) constitués de matériaux de plantes issus de *Knautia arvensis, Arctostaphylos uvaursi* et *Helianthemum canum,* et des extraits de ceux-ci, ii) constitués de matériaux de plantes issus de *Bellis perennis, Eugenia caryophyllata, Olea europaea, Lonicera japonica* et *Symphytum officinale,* et des extraits de ceux-ci, iii) constitués de matériaux de plantes issus de *Carduus pycnocephalus, Populus tremula,* et *Rheum nobile,* et des extraits de ceux-ci, et iv) constitués de matériaux de plantes issus de *Latuca sativa* et *Urtica dioica,* et des extraits de ceux-ci.

9. Utilisation selon la revendication 8, **caractérisée en ce que** la quantité totale des composants appartenant aux groupes i)-iv) est de 20-100% en poids de la quantité totale des composants présents dans l'additif.

10. Utilisation selon les revendications 1-7, **caractérisée en ce que** la quantité totale des composants à administrer va de 0,02 mg à 20 g par jour et kg de poids corporel du ruminant.
